# EUROPEAN PATENT SPECIFICATION

(11) **EP 0 332 421 B1**
(45) Date of publication and mention of the grant of the patent: **22.09.1993**
(21) Application number: 89302305.1
(22) Date of filing: 08.03.1989
(51) Int. Cl.: C07K 15/00, C12P 21/00, C12N 5/00, G01N 33/68, C12N 15/00

(54) **Monoclonal antibodies recognizing human PSTI, hybridomas producing the same, and their preparation and use**
Monoklonale Antikörper, geeignet für den aus der menschlichen Bauchspeicheldrüse sekretierten Trypsin-Inhibitor, Hybridomen, die diese produzieren und ihre Herstellung und Verwendung
Anticorps monoclonaux reconnaissant l'inhibiteur humain de trypsine secrété par le pancréas, hybridomes les produisant, et leur préparation et utilisation

(30) Priority: 08.03.1988 JP 55431/88
(43) Date of publication of application: 13.09.1989
(73) Proprietor: SHIONOGI & CO., LTD., Osaka 541 (JP)
(72) Inventor: Hayashi, Kyozo, Gifu-shi Gifu (JP); Kurobe, Masayuki, Gifu-shi Gifu (JP); Nagata, Kiyoshi, Kobe-Shi Hyogo (JP); Yoshida Nobuo, Nishinomiya-shi Hyogo (JP); Kono, Masao, Ibaraki-shi Osaka (JP)
(74) Representative: Allard, Susan Joyce

(56) References cited:
- EP-A- 0 321 749
- BIOLOGICAL ABSTRACTS, Reports, Reviews and Meetings, vol. 35, no. 55159, Philadelphia, PA (US); J. COLLINS et al., p. 276, no. X 101#
- CLINICA CHIMICA ACTA, vol. 178, 1988, Elsevier Science Publishers BV (BIOMEDICAL DIVISION); M. KUROBE et al., pp. 205-214#
- CHEMICAL ABSTRACTS, vol. 110, no. 23, 05 June 1989, Columbus, OH (US); K. MASAYUKI et al., p. 325, no. 208220u#
- CHEMICAL ABSTRACTS, vol. 89, no. 11, 11 September 1978, Columbus, OH (US); A. EDDELAND et al., pp. 180-181, no. 86370t#

## Description

The present invention relates to, inter alia, anti-human pancreatic secretory trypsin inhibitor monoclonal antibodies, hybridomas which produce such monoclonal antibodies, and a method for the quantitative determination of the human pancreatic secretory trypsin inhibitor with the use of such a monoclonal antibody.

Pancreatic secretory trypsin inhibitor (hereinafter referred to as PSTI) is a trypsin inhibitor which was discovered, for the first time, in the pancreatic juice of cows (Kazal et al., J. Am. Chem. Soc. 70, 3034-3040, 1948). Since then, homologous inhibitors have also been isolated from the pancreatic juice of swine, sheep, humans, dogs, and rats. Digestive enzymes are normally secreted and present in the pancreatic juice in the form of their inactive precursors. It is believed that when the digestive enzymes are abnormally activated in the pancreas for certain reasons, it brings about acute pancreatitis which may be accompanied by not only necrosis of the pancreatic tissue but also dysfunction of other associated organs. PSTI, together with precursors of other enzymes, is secreted into pancreatic juice from the pancreatic acinar cells, and it is believed that when trypsin, which is a trigger of the enzyme activation, is activated from its precursor, PSTI immediately combines with the activated trypsin and prevents the enzyme from exerting its activity until the enzyme reaches the duodenum where the enzyme usually plays its role. Thus, PSTI plays an important role in prevention of acute pancreatitis. Collins et al., Biological Abstracts, Reports, Reviews and Meetings, vol 35, no 55159, pg 276, no X101 describe constructing human PSTI using synthetic genes and site directed mutagenesis which should differentially inhibit trypsin, chymotrypsin and human or porcine elastases without inhibiting other serum proteases. Antisera and monoclonal antibodies to PSTI and to the section site of PSTI are also disclosed in this document.

However, it is also known that the concentration of PSTI remarkably increases in the serum of patients suffering from acute pancreatitis (Kitahara et al., Gall Bladder and Pancreas, 3, 383-388, 1892). According to the recent findings, PSTI acts as an acute phase reactant like α₁-antitrypsin, etc. (Ogawa, Gall Bladder and Pancreas, 7, 1541-1549(1986), and it can also work as a marker for certain tumors (Ogawa, Clinical Pathology, 34, 1229-1235, 1986). Thus, PSTI has an important significance in the area of diagnosis.

As for methods of measuring the concentration of PSTI in serum, enzymatic methods which comprise measuring the afore-mentioned inhibitory activity are difficult due to the co-existence of large amounts of other trypsin inhibitors in the serum. EP-A-0321749 describes monoclonal antibodies specific to human PSTI for use in quantifying proteins in urine and serum, especially for tumour diagnosis.

However, radioimmunoassay (RIA) has already been established for the determination of serum PSTI employing an antagonistic method by the use of rabbit anti-human PSTI serum (Eddeland & Ohlsson, Hoppe-Seyler's Z. Physiol. Chem., 359, 671-675, 1978; Kitahara et al., Biomedical J., 3, 1119-1123, 1979; Japanese Patent Unexamined Publication Nos. 46465/1981, 74653/1981, and 74654/1981), and a kit for the immunoassay is commercially available. According to this method, the serum level of PSTI in patients suffering from acute pancreatitis is remarkably high as compared with that in healthy persons, and the kit has been used in the diagnosis of acute pancreatitis and associated diseases.

However, the antibodies used in the methods disclosed in the above literature are all polyclonal antibodies (rabbit antisera). It is generally recognized that the use of a monoclonal antibody is desirable from the standpoint of specificity, uniformity and steady supply. Given a monoclonal antibody, which is superior to a polyclonal antibody in terms of specificity and affinity, it would be possible to develop a more convenient method of measuring human PSTI with the use of such a monoclonal antibody.

In view of the above circumstances, the present inventors have made strenuous studies and succeeded in obtaining monoclonal antibodies which are very specific to human PSTI and which preferably possess high affinity for PSTI.

Thus, the present invention provides monoclonal antibodies which are obtainable from a hybridoma obtainable from FERM BP 1583, FERM BP 1584 or FERM 1585, and, also, a method of diagnosing acute pancreatitis and related diseases with the use of such monoclonal antibody. The invention also provides hybridomas obtainable from FERM BP 1583, FERM BP 1584 or FERM BP 1585.

In another aspect, the invention includes the use of such antibodies in immunoassay and in in vitro diagnostic procedures.

The anti-human PSTI monoclonal antibodies of the present invention may be produced by hybridomas obtained by fusing human PSTI-immunized mouse lymphocytes with myeloma cells. A process for preparing the monoclonal antibodies of the present invention is detailed below. In general, however, the invention provides a method of producing a hybridoma as defined above or an antibody as defined above comprising immunizing an animal with human PSTI and obtaining antibody-secreting cells therefrom, fusing said cells with myeloma cells, and selecting from the resulting fusion products a fusion product capable of secreting a monoclonal antibody which recognizes human PSTI, and optionally culturing the selected fusion product to produce the desired monoclonal antibody.

First of all, human PSTI is purified for use as an immunogen. Purification of the human PSTI can be conducted by and conventional method.

An experimental animal, for instance a mouse, is then immunized with the immunogen, i.e., the purified human PSTI thus obtained. The immunization can be conducted according to conventional methods. For instance, the antigen dissolved in a physiological saline is mixed with Freund's complete adjuvant to make an emulsion, which is then administered to the animal.

B lymphocytes may be recovered from the animal thus immunized, which are then fused with any one of the permanent myeloma cell lines (for instance, Mouse X63-Ag8, NSI-AG4/1, P3X63-Ag8-Ul, etc.). The fusion may be performed in accordance with the known method reported by Köhler and Milstein (Nature 256, 495-497, 1975). Hybridomas which produce an anti-human PSTI antibody are selected. The selection can be made by examining the presence or absence of the anti-human PSTI antibody in a culture medium in which the hybridomas are grown by a suitable method such as enzyme immunoassay (e.g. ELISA) or radioimmunoassay. The hybridomas which produce anti-human PSTI antibody are cultured until the volume of the culture medium increases to about 2-10 ml, and then freezed, and stored. Hybridomas which produce an antibody having desired properties are selected by examining the antibody secreted in the culture medium. The desired hybridoma thus selected is cloned by a conventional method, such as a limiting dilution method, to establish a cell line. The established cell line is transplanted into an abdominal cavity of an animal of the same species as used for immunization, and ascites containing an antibody with high concentration are obtained The cell line may also be cultured in vitro, and the antibody can be obtained from the culture medium.

The monoclonal antibody thus obtained may be used in immunoassay, after purification if necessary. For instance, the purification may be performed by such conventional methods as ammonium sulfate fractionation, ion exchange chromatography, and protein A column chromatography.

Hybridomas hPSTI-150, 167 and 360, which all produce anti-human PSTI antibodies, and anti-PSTI monoclonal antibodies 150, 167 and 360, which are respectively produced by the above hybridomas, were obtained according to the methods described above.

These hybridoma cell lines have been deposited under the Budapest Treaty with the Fermentation Research Institute, Agency of the Industrial Science & Technology, Higashi 1-1-3, Tsukuba City, Ibaraki Prefecture, since Dec. 3, 1987, under the following accession numbers:
Hybridoma hPSTI-150:
Bikoken Joki No. 1585 (FERM BP-1585)
Hybridoma hPSTI-167:
Bikoken Joki No. 1583 (FERM BP-1583)
Hybridoma hPSTI-360:
Bikoken Joki No. 1584 (FERM BP-1584)
Monoclonal antibodies 150, 167 and 360 have the following properties:
(1) The immunoglobulin class and subclass of monoclonal antibody 150 is IgG₂ₐ, and antibodies 167 and 360 are each IgG₁, and the L chains of these antibodies are all κ type.
(2) The binding constants of monoclonal antibodies 150, 167 and 360 are 4.9 x 10⁸ M⁻¹, 3.7 x 10⁹ M⁻¹, and 1.4 x 10⁹ M⁻¹ respectively. All of these antibodies show high affinity.
(3) Monoclonal antibody 150 does not neutralize the trypsin-inhibiting activity of PSTI, but 167 and 360 neutralize the inhibiting activity. That is to say, monoclonal antibody 150 has specificity different from those of monoclonal antibodies 167 and 360.

Thus, monoclonal antibodies of the present invention may be very specific to human PSTI, and usually possess high affinity with the same, and therefore, they are excellent antibodies for use in the immunological measurement of human PSTI.

With the use of the monoclonal antibodies of the present invention, PSTI can be immunologically measured, for instance, in the manner as detailed below.

Human serum PSTI can be measured by radioimmunoassay employing a competitive method, which is characterized by the use of a monoclonal antibody of the present invention together with human PSTI labeled with a radioisotope such as ¹²⁵I and ¹³¹I. That is to say, the immunoassay itself is performed by a known method, but a predetermined amount of the monoclonal antibody of the invention is allowed to react competitively with a predetermined amount of ¹²⁵I-labeled PSTI (¹²⁵I is introduced into Tyr residue of PSTI by a known method such as the chloramine T method) and a standard human PSTI or a serum sample. Then, by a double-antibody method or a polyethylene glycol method, the ¹²⁵I-labeled PSTI bound to the antibody (such PSTI is hereinafter referred to as "bound") is separated from the free ¹²⁵I-labeled PSTI (such PSTI is hereinafter referred to as "free"). After that, radioactivity of the bound PSTI or free PSTI is measured. Comparison of the radioactivity of the standard PSTI with that of serum sample gives the concentration of serum PSTI.

Human serum PSTI can also be measured by a competitive enzyme immunoassay which comprises the use of the monoclonal antibody of the present invention together with human PSTI labeled with an enzyme such as peroxidase and β-D-galactosidase. That is, human PSTI labeled with an enzyme can be used instead of the PSTI labeled with ¹²⁵I employed in the above-mentioned radioimmunoassay. Enzyme activity can be measured by a colorimetric method using a color-developing agent such as o-phenylenediamine, or fluorometry using a fluorescent substrate such as p-hydroxyphenylpropionic acid, when peroxidase is employed as the enzyme.

Enzyme immunoassay can be conducted on a homogeneous system in which the separation of the "bound" from the "free" is not required. For instance, in the method known by a commercial term of EMIT® (Enzyme Multiplied Immunoassay Technique), the amount of the "bound" can be estimated from the decrease of the enzyme activity of the bound enzyme-labeled human PSTI as compared with that of the "free", said decrease of enzyme activity being caused by steric hindrance due to the attached antibody or a structural change in the active center of the enzyme. Enzyme immunoassay can also be applied to another homogeneous system such as enzyme channeling immunoassay.

Chemiluminescent immunoassay or bioluminescent immunoassay can also be effectal by the use of a monoclonal antibody of the present invention, wherein human PSTI labeled with a chemiluminescent substance such as acridinium ester and isoluminol, or with a bioluminescent substance such as luciferin, is employed. That is to say, the measurement may be carried out by following the afore-mentioned procedure with the exception that human PSTI labeled with a luminescent substance is used instead of the ¹²⁵I-labeled PSTI in the afore-mentioned radioimmunoassay. Intensity of the luminescence of the "bound" or the "free" is measured by a luminometer.

Moreover, fluoroimmunoassay may be conducted, wherein human PSTI labeled with a fluorescent substance such as fluorescein isothiocyanate is employed.

Non-competitive immunoassay may also be conducted, wherein two different monoclonal antibodies of the present invention, which recognize different sites of the human PSTI molecule, are used.

One of various possible methods employing the two different monoclonal antibodies is an immunoradiometric assay in which one of the monoclonal antibodies is immobilized and the other is labeled with a radioisotope such as ¹²⁵I. That is, one of the monoclonal antibodies is adsorbed or chemically bound to a solid phase such as polystyrene beads, and the other is labeled with a radioisotope such as ¹²⁵I. A standard human PSTI or a serum sample is then contacted with the immobilized antibody, after which the ¹²⁵I-labeled antibody is added and allowed to bind to the human PSTI captured on the solid phase. The supernatant (containing the "free") is then removed, and the radioactivity of the "bound" is measured. Comparison of the radioactivity of the standard human PSTI with that of the serum sample gives the PSTI concentration in the serum sample.

In addition to the "two-step" procedure as mentioned above, the assay can be carried out by a single step, wherein the standard human PSTI or the serum sample is added to the solid phase together with ¹²⁵I-labeled antibody at the same time, the supernatant is then removed, and the radioactivity of the "bound" is measured.

In place of the monoclonal antibody labeled with a radioisotope, an antibody labeled with an enzyme can be employed in the above assay. As the enzyme, there may be mentioned peroxidase and β-D-galactosidase. More particularly, one of the monoclonal antibodies of the present invention is adsorbed or chemically bound to the solid phase such as polystyrene beads, and another antibody is labeled with an enzyme such as peroxidase and β-D-galactosidase. The former can be labeled by means of the maleimide method or periodic acid oxidation method, while the latter can be labeled by the maleimide method or glutaraldehyde method.

A standard human PSTI or a serum sample is then contacted with the immobilized antibody, after which the enzyme-labeled antibody is added and allowed to bind to the human PSTI captured on the solid phase. The supernatant (free) is then removed, and the radioactivity of the "bound" is measured. Comparison of the radioactivity of the standard human PSTI with that of the serum sample gives the PSTI concentration of the serum sample. The single step procedure as mentioned above can also be employed here.

The enzyme activity of peroxidase or β-D-galactosidase can be measured by a colorimetric method using a color-developing agent such as o-phenylenediamine or o-nitrophenyl-β-D-galactoside, or fluorometry using a fluorescent substrate such as p-hydroxyphenylpropionic acid or 4-methylumbelliferyl-β-D-galactoside.

Non-competitive enzyme immunoassay is applicable to a homogeneous system which does not require separation of the "bound" from the "free".

Enzyme-enhanced immunoassay is employable for the measurement of human serum PSTI, wherein one monoclonal antibody of the invention labeled with β-D-galactosidase and another antibody labeled by succinylation are used together with o-phynyl-β-D-galactoside bound to dextran. More particularly, one of the monoclonal antibodies of the invention is labeled with β-D-galactosidase by the maleimide method or the like , and another antibody of the invention is succinylated by succinic anhydride. These modified antibodies are then contacted with a standard human PSTI or a serum sample so as to allow binding to the human PSTI molecule. To the mixture is added o-phenyl-β-D-galactoside attached to dextran, and turbidity is measured with a nephelometer. Comparison of the turbidity of the standard human PSTI with that of the serum sample provides the concentration of PSTI in the sample.

Immunoluminometric assay which employs one monoclonal antibody immobilized on a solid phase and another antibody labeled with a luminescent substance such as acridinium ester may also be employable. More particularly, one of the monoclonal antibodies of the invention is adsorbed or chemically bound to a solid phase such as polystyrene beads, and another antibody is labeled with an acridinium ester such as 4-(2-succinimidyloxycarboxyethyl)phenyl-10-methylacridinium- 9-carboxylate fluorosulfonate. The labeling can be effected by adding an antibody-containing phosphate buffer, pH 8.0, to a solution of the afore-mentioned acridinium ester dissolved in DMF.

A standard human PSTI or a serum sample is then contacted with the immobilized antibody, after which the supernatant (free) is eliminated and the intensity of the luminescence of the "bound" is measured by a luminometer. Comparison of the intensity of the luminescence on the standard human PSTI with that on the serum sample gives the PSTI concentration of the serum sample.

Non-competitive immunoassay which employs one of the monoclonal antibodies of the present invention together with an anti-human PSTI polyclonal antibody prepared by sensitizing a rabbit or the like can also be useful. Either the monoclonal antibody or the polyclonal antibody is immobilized on a solid phase, and the other is labeled with a radioisotope, an enzyme or a luminescent substance. Measurement is carried out by following the procedures mentioned above.

Non-competitive immunoassay employing two different antibodies further includes following special techniques.

A monoclonal antibody of the invention is adsorbed or chemically bound to a solid phase such as polystyrene balls, while a goat anti-rabbit IgG antibody is labeled with the afore-mentioned radioisotope, an enzyme or a luminescent substance. A standard human PSTI or a serum sample is contacted with the antibody on the solid phase, followed by the addition of a rabbit anti-human PSTI polyclonal antibody (antiserum) so that the polyclonal antibody may combine with the human PSTI captured on the solid phase. The goat anti-rabbit IgG antibody labeled with an enzyme and the like is added to bind to the rabbit anti-human PSTI polyclonal antibody, and the enzyme activity of the "bound" is measured in the same manner as mentioned above.

Various immunological assays employing the monoclonal antibodies of the present invention are illustrated in the above. However, the scope of the invention is not intended to be limited thereby.
In the accompanying drawings:
Fig. 1 shows a standard curve for immunoradiometric assay which employs the monoclonal antibodies of the invention.
Fig. 2 illustrates a dilution curve of the monoclonal antibodies of the invention.
Fig. 3 shows the cross reactivity of monoclonal antibodies of the invention. Figs. 3A, 3B and 3C respectively represent the cross reactivity of monoclonal antibodies 167, 150 and 360.
Fig. 4 represents an elution curve of ¹²⁵I-labeled PSTI.
Fig. 5 illustrates a standard curve for radioimmunoassay which employs the antibody 167 of the invention and a standard curve prepared by PSTI Test Shionogi.
Fig. 6 shows a dilution curve of human serum by radioimmunoassay with the use of the antibody 167.
Fig. 7 illustrates the correlation between the data obtained by radioimmunoassay of the invention with the use of the antibody 167 and the data obtained by PSTI Test Shionogi.
Fig. 8 shows an elution curve of ¹²⁵I-labeled anti-PSTI antibody.
Fig. 9 represents a standard curve for radioimmunometric assay by sandwich method with the use of the antibody 167.
Fig. 10 shows a standard curve for an enzyme immunoassay by one-step method.
Fig. 11 shows a standard curve for an enzyme immunoassay by two-step method.

### Example 1 Preparation of hybridomas producing an anti-human PSTI monoclonal antibody, and preparation of the monoclonal antibodies

### (1) Preparation of antigen

Human PSTI used as an immunogen in the following immunization step was isolated and purified from human pancreatic juice in accordance with the method of Kikuchi et al. (J. Biochemistry 98, 687-694, 1985).

### (2) Immunization

A solution of human PSTI in a physiological saline (0.5 or 0.1 mg/ml) was mixed with an equivalent amount of Freund's complete adjuvant to prepare an emulsion. The emulsion (0.2 ml /animal) was subcutaneously injected 4 times in the back of BALB/c mice (females, 5 weeks of age) at intervals of 4 weeks. The mice each received 50 µg or 10 µg of PSTI. Ten days after the 4th immunization, 200 µl of a physiological saline containing 50 µg of human PSTI was boostered intraperitoneally.

### (3) Cell fusion

Three days after the booster immunization, the mouse spleen was taken out, and the cells were placed in a 0.17 M ammonium chloride solution for 5 min. under cooling with ice so as to destroy the erythrocytes. The remaining cells were suspended in RPMI1640 medium, which was used as splenic lymphocytes for fusion.

Subsequently, 6.3 x 10⁷ (or 3.5 x 10⁷) cells of 8-azaguanine-resistant myeloma cells (NS-1) suspended in RPMI1640 medium were mixed with 1.9 x 10⁸ (or 3.5 x 10⁸) cells of the splenic lymphocytes. After centrifugation (1,000 rpm, 10 min.) of the mixture, the supernatant was removed. To the precipitated cells was added 1.0 ml of 50% polyethylene glycol (molecular weight 4,000, Merck) in RPMI1640 medium over a period of 1 min., while stirring with the tip of a pipet, and the mixture was further stirred for 1.5 min. Two ml of RPMI1640 was then added over a period of 2 min., followed by the addition of another 2 ml of the medium over a period of 1 min. with stirring. In addition, 18 ml of RPMI1640 was dropwise and slowly added with gentle stirring. After centrifugation (1,000 rpm, 10 min.) of the mixture, the supernatant was removed, and the precipitated cells were suspended in 90 ml (or 160 ml) of HAT medium (20% FCS-RPMI1640 medium containing 1 x 10⁻⁴ M hypoxanthine, 4 x 10⁻⁷ M aminopterin, and 1.6 x 10⁻⁵ M thymidine). The suspension was distributed into 9 (or 16) pieces of 96-well culture plate (Costar) at 0.1 ml per well.

In the cell culture plates were placed beforehand a suspension of mouse spleen cells in HAT medium as a vegetative cell at the rate of 1 x 10⁵ cells/0.1 ml/well. Thereafter, half of the HAT medium was replaced with fresh medium at two or three days intervals. The growth of hybridomas was observed in about 10 days. There were a total of 629 wells (25%) in which hybridomas had grown.

### (4) Frozen storage of hybridomas

All of the hybridomas were further grown in HT medium, which corresponds to the above-mentioned HAT medium free of aminopterin, up to about 2 ml of the medium. The hybridomas were then suspended in 0.5 ml of a freezing medium (fetal calf serum containing 10% DMSO), and stored at -80°C. Supernatant of each culture was separately stored to examine the properties of the antibodies produced.

### (5) Selection of hybridomas (Part I)

Preliminary ELISA which employed 96-well microplates (0.1 µg/well) coated with PSTI showed that most of the hybridomas produced anti-PSTI antibodies. Therefore, in order to select desired strains producing antibodies having high affinity, which appear suitable for immunoassay, a radioimmunoassay was performed.

One hundred µl of the above-mentioned culture supernatant and 100 µl of each of 1:10 and 1:100 dilutions of the supernatant were separately mixed with 100 µl of ¹²⁵I-labeled human PSTI (mentioned in Example 3) and 50 µl of a buffer solution for assay (0.01 M phosphate buffer, pH 7.4, containing 0.9% sodium chloride, 0.1% sodium azide, 1 mM tetrasodium ethylenediaminetetraacetate, and 0.5% bovine serum albumin) containing 2% bovine γ-globulin and the resultant mixtures were incubated for 1 hr. at 37°C. To each of the above mixtures were added 100 µl of a phosphate-buffered physiological saline containing 2% bovine γ-globulin and 500 µl of 20% polyethylene glycol 6000. The mixtures were stirred immediately, and after centrifugation (3,000 rpm, 20 min.), the supernatants were removed by suction, and the radioactivity of the resultant precipitate in each sample was measured, which reflects the amount of the ¹²⁵I-labeled human PSTI bound to the antibody.
On the other hand, the same procedure as mentioned above was repeated except that 50 µl of the buffer solution for assay containing 40 ng/ml of human PSTI was added instead of the afore-mentioned buffer solution for assay, and the decrease in the radioactivity of ¹²⁵I-labeled human PSTI in the precipitate was examined. That is, the extent to which the ¹²⁵I-labeled human PSTI was replaced by the human PSTI which had been added beforehand was examined. The culture supernatant which showed high degree of replacement was regarded as one containing an antibody showing high affinity. In this way, 21 strains were selected on the following criteria: where the 1:10 dilution of the supernatant is used, the amount of "bound" PSTI against the total amount of PSTI (B/T) must be 20% or more; and where the 1:10 or 1:100 dilution is used, 10% or more of "bound" labeled PSTI must be replaced by PSTI.

### (6) Selection of hybridomas (Part II)

An RIA similar to that described above was repeated using the dilutions of the supernatant of the 21 hybridoma cultures selected in (5), after the supernatants were adjusted to B/T=25%. This time, 50 µl of the standard PSTI solution (0, 2, 8, 32, 125, or 500 ng/ml) was added instead of 50 µl of the buffer solution for assay in order to obtain the standard curve of PSTI. Experiment was done in the same manner as in (5), and the standard curve was prepared based on the amount of the "bound" labeled PSTI obtained. In addition, the binding constant between the antibody contained in each supernatant and PSTI was calculated in accordance with the known Scatchard plot method (Ann. N.Y. Acad. Sci. 51, 660 (1949). Only those hybridomas which showed a binding constant of 1 x 10⁸M⁻¹ or higher were selected, which were 7 strains designated as hybridoma hPSTI-150, 167, 179, 360, 384, 432, and 622. The binding constants of the antibodies were 4.9 x 10⁸, 3.7 x 10⁹, 4.8 x 10⁸, 1.4 x 10⁹, 6.0 x 10⁹, 5.1 x 10⁸, and 1.7 x 10⁸M⁻¹, respectively.

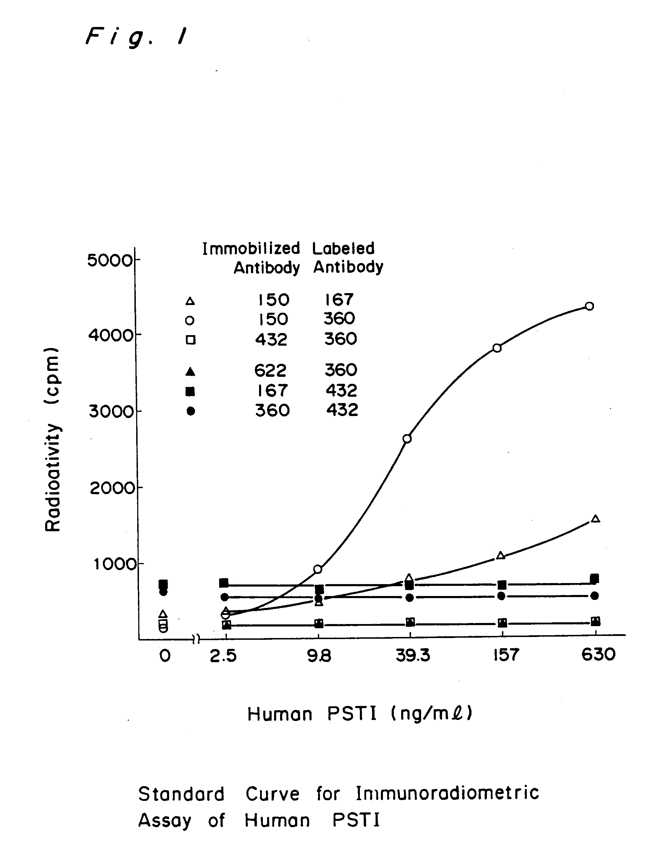

Of these strains, hybridoma hPSTI-167 which showed high affinity and high production of antibody was selected. In addition, immunoradiometric assay, which will hereinafter be described in detail, revealed that a combination use of additional strains, i.e., hybridomas hPSTI-150 and 360 were suitable for the sandwich method (See Fig. 1).

### (7) Cloning

The hybridomas selected above were thawed and cloned by limiting dilution. The hybridomas were cultured in a 96-well cell culture plate at a concentration of 0.5 cell/200 µl/well, and the above-mentioned radioimmunoassay was performed on the supernatant of the wells in which the hybridomas had grown to give a single colony. Hybridomas which produced anti-human PSTI antibody were further cultured. In such a way, hybridoma cell lines hPSTI-150, 167 and 360, which produce anti-human PSTI monoclonal antibodies, were established.

### (8) Preparation of antibody-containing ascites

The hybridoma thus established was transplanted into the abdominal cavity of a mouse so as to produce ascites containing a high concentration of the desired monoclonal antibody.

A suspension of about 3 x 10⁶ cells of hybridoma in a phosphate-buffered physiological saline was injected into the abdominal cavity of a mouse (BALB/c, female; 0.5 ml of pristane had been injected intraperitoneally 10 days before), and ascites was collected at 1 to 3 weeks intervals after injection. After separation of the cells by centrifugation, 0.1 % of sodium azide was added to the supernatant, which was frozen and stored. In this way, ascites 150A, 167A, and 360A containing high concentration of monoclonal antibodies were obtained, which were produced by the use of hybridomas hPSTI-150, 167, and 360, respectively.

### (9) Purification of monoclonal antibody

Antibodies were isolated and purified from the ascites 150A, 167A, and 360A by Affigel Protein A MAPS-II Kit (Bio-Rad).

One ml of each of the ascites was purified using 2 ml of the gel according to the procedures indicated in the kit, to obtain about 7 mg of antibody from ascites 150A, about 6 mg from ascites 167A, and about 6 mg from ascites 360A. The antibodies were subjected to SDS-polyacrylamide electrophoresis to examine their purity. The purified antibodies, after being reduced with 2-mercaptoethanol, were subjected to an electrophoresis on 12.5 % SDS gel. There were observed two bands at about 53,000 (MW) and about 26,000 (MW), the former being H-chain and the latter being L-chain.

### Example 2 Properties of monoclonal antibodies

### (1) Determination of class & subclass

Determination of class & subclass of the immunoglobulins produced by the hybridomas was conducted by the afore-mentioned ELISA using the rabbit IgG antibodies specific for each of classes & subclasses of mouse immunoglobulin and peroxidase-labeled goat anti-rabbit antibody (MonoAb-ID EIA Kit, Zymed Laboratories). Development of color was observed in ascites 150A when anti-γ₂ₐ antibody and anti-κ antibody were used, and in ascites 167A and 360A when anti-γ₁ antibody and anti-κ antibody were used, which showed that the monoclonal antibody 150 belongs to IgG₂ₐ, and the monoclonal antibodies 167 and 360 to IgG₁, and that L-chain is each κ type.

### (2) Immunological properties of monoclonal antibodies

Immunological properties of ascites 150A, 167A and 360A, which contain anti-PSTI monoclonal antibodies mentioned in the afore-mentioned Example 1-(8), were determined by radioimmunoassay which will be described in subsequent Example.

### Antibody titer

Antibody solutions were serially 4-fold diluted (1:2000-1:2048000). Each of the diluted solutions (500 µl) was charged in a polystyrene tube and mixed with 100 µl of ¹²⁵I-labeled PSTI solution (5.5 x 10⁵ dpm/ml) and 50 µl of a buffer solution for measurement, which contains no standard PSTI, and the mixture was incubated for 2hr. at 37°C. Antibody titer was measured on the resultant mixtures in accordance with the method previously described.

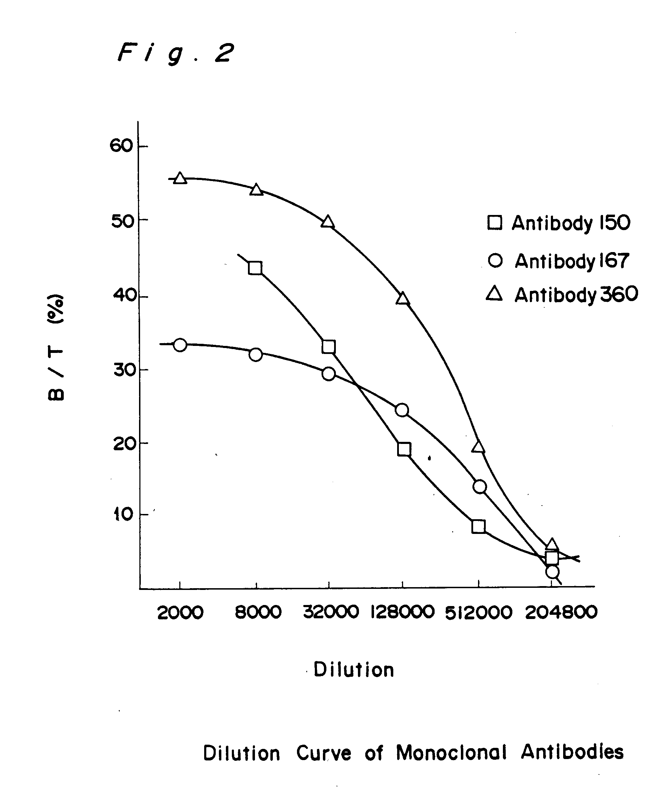

The ratio (B/T%) of the count (B) of each of the diluted antibody solutions against the count (T) of the ¹²⁵I-labeled PSTI added was plotted at the lin (linear) side, while dilution multiple of the antibody was plotted at the log side, and thereby a dilution curve was obtained. The dilution multiple of the antibody required for 20% of 55000 dpm of ¹²⁵I-labeled PSTI to bind to the antibody represents the antibody titer. The antibody titers of the monoclonal antibodies 150, 167 and 360 were 100000, 150000, and 360000 respectively. The dilution curve of each antibody is shown in Fig. 2.

### Affinity

The binding constants of the ascites 150A, 167A and 360A with PSTI, which were calculated by the known plot method of Scatchard [Ann. N.Y. Acad. Sci., 51, 660 (1949)], were 4.9 x 10⁸M⁻¹, 3.7 x 10⁹M⁻¹, and 1.4 x 10⁹M⁻¹ respectively.

### Cross reactivity

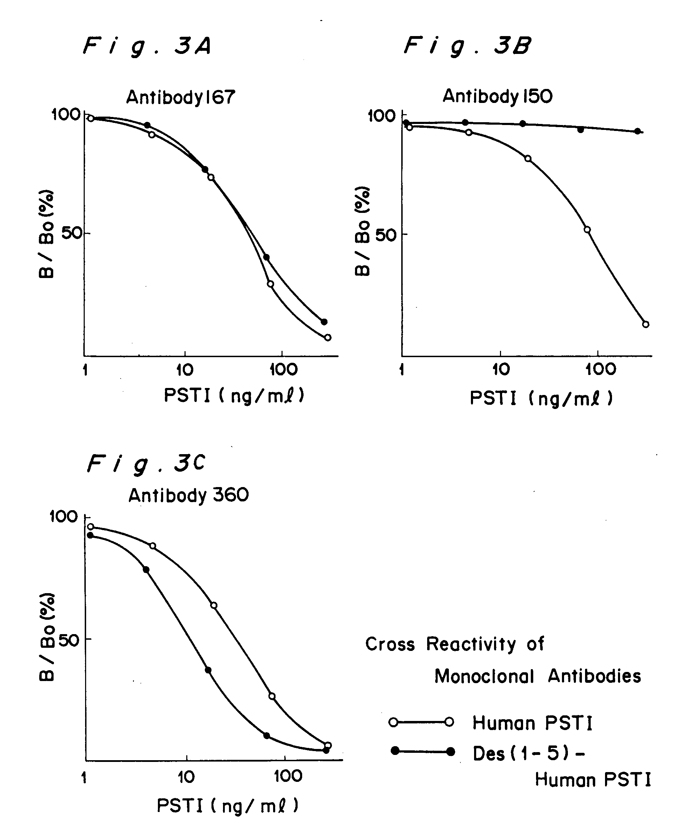

Cross reactivity of 150A, 167A and 360A with des(1-5)-PSTI is shown in Fig. 3. The cross reactivity was <5, 90, and 290% respectively, when the reactivity with PSTI is taken as 100%.

None of 150A, 167A and 360A showed cross reactivity at all with an animal serum.

### Neutralization of trypsin-inhibiting activity of PSTI with monoclonal antibodies

About 15 µg of IgG isolated from ascites 150A, 167A, or 360A was mixed with 0.9 ml of a 0.1 M tris hydrochloric acid buffer (pH 8.0) containing 0.5 µg of PSTI, and the mixture was warmed at 25°C for 30 min., after which 1 mM hydrochloric acid containing 2.3 µg of bovine trypsin and 0.1 ml of a 20 mM aqueous solution of calcium chloride were added thereto and mixed. The mixture was warmed at 25°C for 15 min., after which 1 ml of a 0.1 M tris hydrochloric acid buffer containing 868 µg of benzoyl-L-arginine-p-nitroanilide hydrochloride was added, and the mixture was further warmed at 25°C for 10 min. Then, 0.5 ml of a 30% aqueous solution of acetic acid was added to stop the enzyme reaction. The residual enzyme activity can be measured by determination of absorbance of the reaction solution at 410 nm. The absorbance in the reaction system containing neither PSTI nor IgG was taken as a total activity or an intact activity of the enzyme. A reaction system free of IgG but containing PSTI and trypsin showed the residual activity of 20%. The presence of IgG derived from ascites 167A or 360A showed the residual activity of 80%. On the other hand, the presence of IgG derived from ascites 150A showed the residual activity of 20%.

The above test results indicate that monoclonal antibodies 167 and 360 produced by hybridomas hPSTI-167 and 360 neutralize the trypsin-inhibiting activity of PSTI, but monoclonal antibody 150 does not neutralize the inhibiting activity. That is to say, monoclonal antibodies 167 and 360 have a specificity different from that of monoclonal antibody 150.

### Example 3 Radioimmunoassay

### (1) Preparation of ¹²⁵I-labeled PSTI

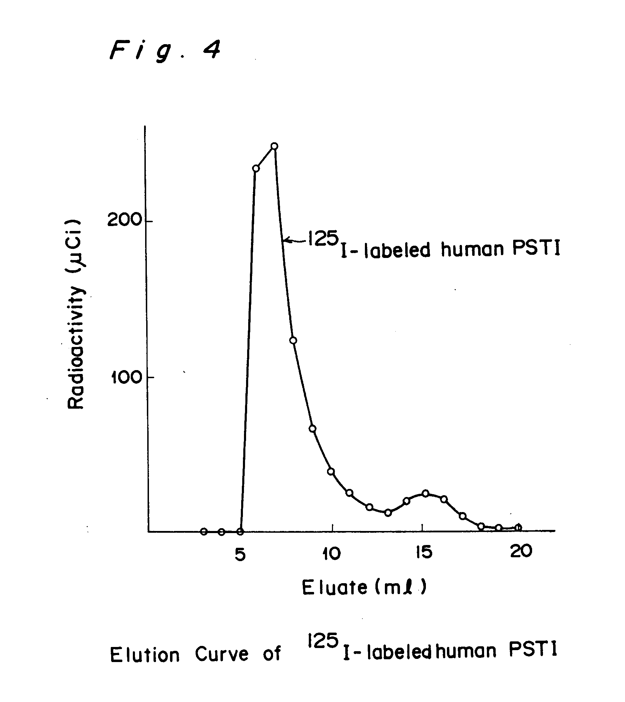

To 10 µl of a 0.1 M phosphate buffer (pH 7.4) containing 5 µg of PSTI were added 50 µl of a 0.5 M phosphate buffer and 10 µl of Na¹²⁵I (Amersham, 100 mCi/ml), and admixed. To the mixture, 10 µl of 0.2% chloramine T was added and stirred for 30 seconds at room temperature. Ten µl of 1% sodium pyrosulfite was then added thereto, and mixed, after which 10 µl of each of 10% sodium iodide and 1% bovine serum albumin was added. The reaction mixture was purified by gel filtration [Sephadex G-25 (Fine, 0.9 x 25 cm, Pharmacia), 0.1 M phosphate buffer (pH 7.0)] to obtain ¹²⁵I-labeled PSTI with a specific radioactivity of 200 µCi/µg. The chromatogram (elution curve) of the gel filtration is shown in Fig. 4.

### (2) Radioimmunoassay of PSTI

### Method of measurement

Dilution and preparation of the reagents employed in this method were always performed with the use of 0.01 M phosphate buffer (pH 7.4) containing 0.9% sodium chloride, 0.1% sodium azide, 1 mM tetrasodium ethylenediaminetetraacetate, and 0.5% bovine serum albumin.

Fifty µl of the standard PSTI solution (0, 2, 5, 10, 50, 150, 500 ng/ml) or a serum sample was charged in a polystyrene tube. To the tube were added 100 µl of the afore-mentioned ¹²⁵I-labeled PSTI solution (5.5 x 10⁵ dpm/ml) and 500 µl of the dilution (1:150,000) of ascites 167A, and the mixture was incubated at 37°C for 2 hr. One hundred µl of a suspension of goat anti-mouse IgG antibody immobilized on a solid phase (1 mg/ml, Bio-Rad) was added, and the mixture was incubated at 37°C for 30 min. The mixture was then centrifuged (1500 x g, 10 min.), and the supernatant was removed by suction, and the radioactivity of the precipitate was measured by means of a gamma-ray scintillation counter.

### Standard curve

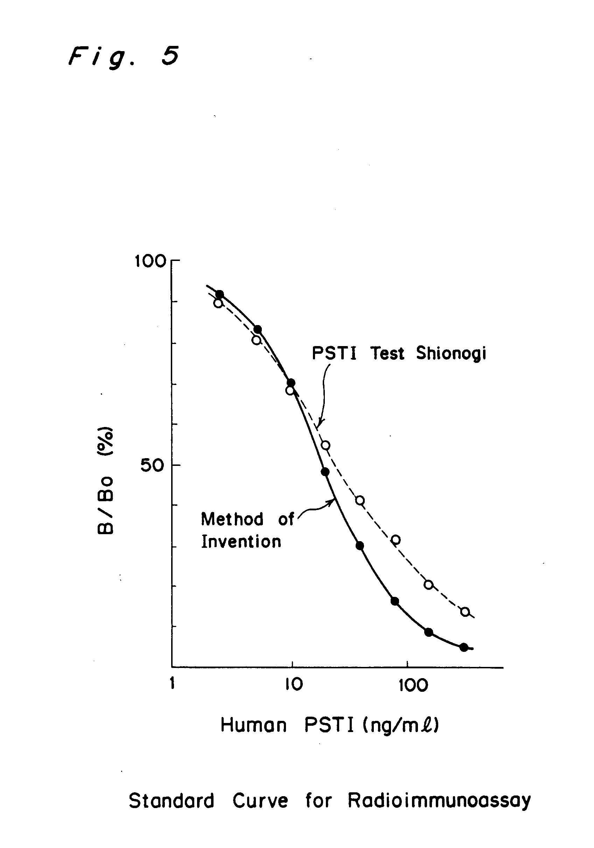

The standard curve was prepared by plotting the concentration of the standard PSTI at the log side, and the ratio (B/Bo%) of the count (B) of respective standard PSTIs against the count (Bo) of zero(0) ng/ml of PSTI at the lin side. The concentration of PSTI in a given serum was measured from the B/Bo% determined on the serum. The standard curve prepared by the method mentioned above and a conventional standard curve attached to PSTI Test Shionogi (Shionogi & Co., Ltd.), which is commercially available as an in vitro diagnostic agents kit for acute pancreatitis or the like, are as shown in Fig. 5.

### Recovery test of serum PSTI

The standard PSTI was added to the serum of healthy persons, and the recovery of the PSTI was measured by the method mentioned above. The recovery rate of the PSTI is shown in Table 1. An average recovery rate was 102.2 ± 6.8%.

**Table 1**

| Recovery Test of Serum PSTI | | | | |
|---|---|---|---|---|
| PSTI Added (ng/ml) | Serum 1 | | Serum 2 | |
| | Found (ng/ml) | Recovery Rate (%) | Found (ng/ml) | Recovery Rate (%) |
| 0 | 11.2 | | 12.7 | |
| 3.9 | 15.3 | 101 | 15.6 | 94.0 |
| 7.8 | 20.9 | 110 | 22.3 | 109 |
| 15.6 | 29.5 | 110 | 26.5 | 93.6 |
| 31.2 | 42.0 | 99.1 | 44.2 | 101 |

### Dilution curve of human serum

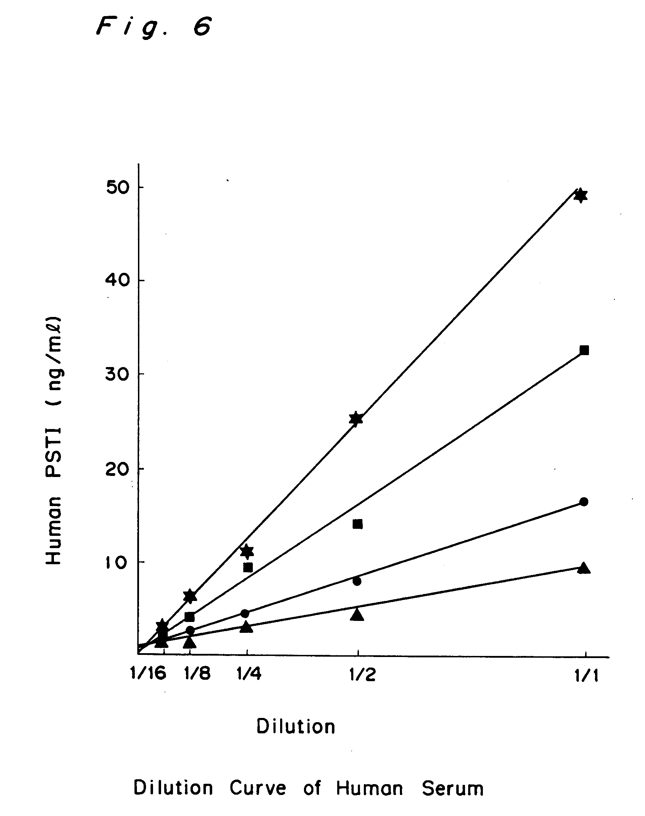

The concentration of PSTI was measured on various serum samples which were prepared by serial 2-fold dilution of original sera obtained from healthy persons and patients suffering from pancreatic diseases. The dilution rate was plotted at the log side, and the B/Bo% at the lin side. The curve obtained is shown in Fig. 6.

### Concentration of PSTI in serum of healthy persons

The concentration of PSTI in serum was measured on 10 healthy persons according to the afore-mentioned method. Table 2 shows the test results with an average concentration of 9.4 ± 1.9 ng/ml. The same serum samples were measured with the afore-mentioned PSTI Test Shionogi, which gave the test result as shown in Table 2, with an average concentration of 8.4 ± 2.3 ng/ml.

**Table 2**

| Concentration of PSTI in Sera of Healthy Persons | | |
|---|---|---|
| Serum | PSTI Concentration (ng/ml) | |
| | Method of Invention | PSTI Test Shionogi |
| 1 | 11.2 | 11.6 |
| 2 | 12.7 | 11.5 |
| 3 | 10.6 | 9.5 |
| 4 | 8.5 | 7.7 |
| 5 | 10.3 | 9.1 |
| 6 | 9.6 | 8.3 |
| 7 | 8.5 | 8.4 |
| 8 | 6.9 | 6.0 |
| 9 | 6.7 | 4.1 |
| 10 | 8.5 | 7.4 |
| Average Concentration | 9.4±1.9 | 8.4±2.3 |

### Concentration of PSTI in serum of patients suffering from acute pancreatitis

The concentration of PSTI in serum of patients suffering from acute pancreatitis is shown in Table 3, which shows significantly high concentration of PSTI as compared with those contained in serum of healthy persons.

**Table 3**

| Concentration of PSTI in Sera of Patients Suffering from Acute Pancreatitis | |
|---|---|
| Serum | Concentration of PSTI (ng/ml) |
| 1 | 320 |
| 2 | 30 |
| 3 | 83 |
| 4 | 61 |
| 5 | 56 |
| 6 | 28 |
| 7 | 61 |
| 8 | 18 |

### Comparison with the test results obtained by PSTI Test Shionogi

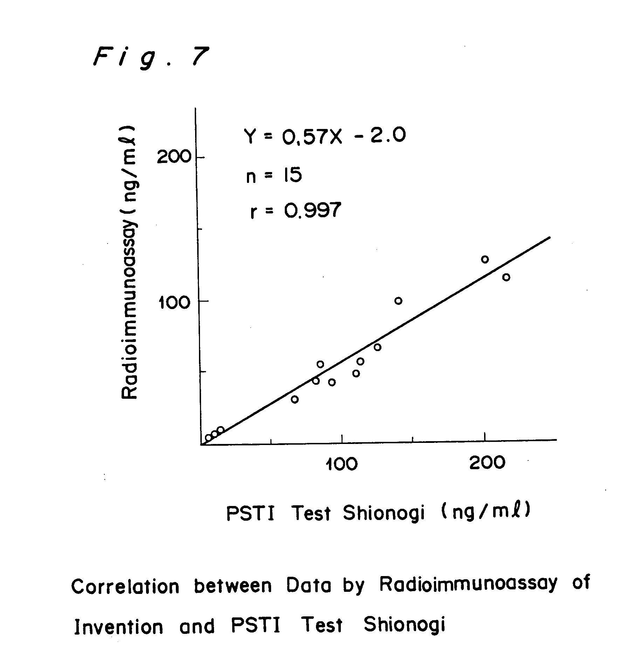

A total of 15 samples of human serum were measured by the method of the present invention and the aforementioned PSTI Test Shionogi. Fig. 7 shows the test results. A good correlation was observed between both methods.

### (3) Immunoradiometric assay of PSTI

### Purification of ascites

The ascites obtained by the use of the afore-mentioned hybridomas hPSTI-150 and 360 were purified by means of Affigel Protein A MAPS-II Kit (Bio-Rad) in the manner as follows.

One ml of the ascites was added to 1 ml of a binding buffer, and the mixture was added to the Affigel Protein A column ( 0.7 cm φ x 5.5 cm) equilibrated with the binding buffer. The column was then washed with 30 ml of the binding buffer and eluted with 10 ml of an eluting buffer. The eluate was collected in 1 ml portions, and the fractions showing absorbance of 0.1 or more at 280 nm were collected and dialyzed against distilled water to obtain IgG fraction.

### Preparation of antibody immobilized on a solid phase

Fifty pieces of polystyrene bead (Sekisui Chemical Co., Ltd., 6.4 mm in diameter) were immersed in 50 ml of a 0.05 M phosphate buffer (pH 7.5) containing 0.35 mg of the anti-PSTI monoclonal antibody 150 obtained in the aforementioned Example 1 (9), and allowed to stand for 16 hr. at room temperature. The beads were then thoroughly washed with 0.01 M phosphate buffer, pH 7.4, containing 0.2% bovine serum albumin, 5 mM tetrasodium ethylenediaminetetraacetate, 0.15M sodium chloride, and 0.1% sodium azide (Buffer A). After that, the beads were stored in Buffer A at 4°C.

### Preparation of ¹²⁵I-labeled PSTI antibody

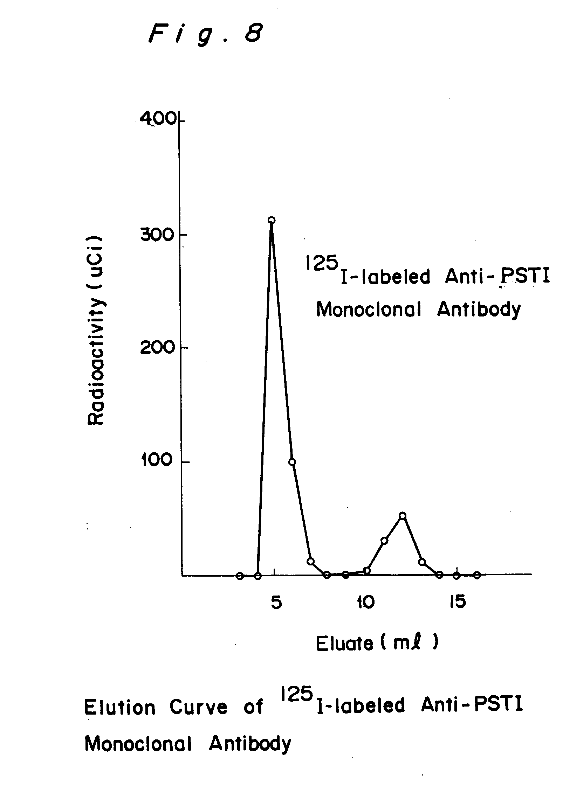

The anti-PSTI monoclonal antibody 360 (5 µg) obtained in the afore-mentioned Example 1 (9) was mixed with 50 µl of 0.5 M phosphate buffer (pH 7.5) and 1 mCi of Na¹²⁵I (Amersham). Ten µl of 0.2% chloramine T was added thereto, and the mixture was stirred for 45 seconds at room temperature. To the mixture, 10 µl of 1% sodium pyrosulfite was added and mixed, and thereafter, 10 µl of each of 10% potassium iodide and 1% bovine serum albumin were added. The reaction mixture was subjected to gel filtration [Sephadex G-25 (Fine, 0.75 cm φ x 25 cm, Pharmacia); 0.1 M phosphate buffer, pH 7.4]. The elution curve obtained is shown in Fig. 8.

### Sandwich method

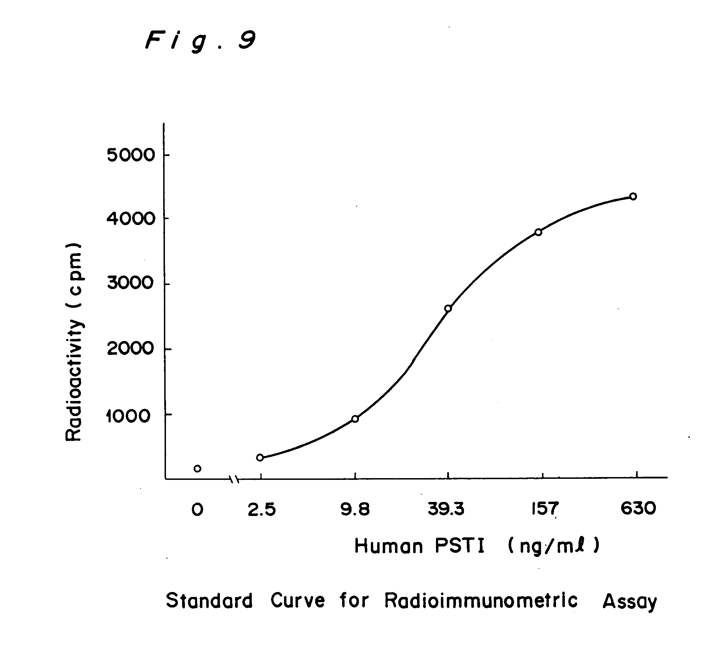

A piece of the afore-mentioned bead which carries the immobilized antibody was placed in a polystyrene tube. Two hundreds µl of a standard PSTI solution was added thereto and the mixture was incubated at 37°C for 2 hr. The solution was removed by suction, and the bead was washed twice with each 2 ml of Buffer A. After that, 200 µl of a solution of ¹²⁵I-labeled PSTI antibody was added, which was then incubated at 37°C for 2 hr. The solution was removed by suction, and the bead was washed twice with each 2 ml of Buffer A, and the radioactivity on the solid phase was measured with a gamma-ray scintillation counter. By plotting the concentration of the standard PSTI at the abscissa and the radioactivity at the ordinate, a standard curve shown in Fig. 9 was obtained.

### Exmple 4 Enzyme immunoassay

### Preparation of anti-PSTI antibody Fab′ fragment

The anti-PSTI monoclonal antibody 360 (4.4 mg) obtained in the afore-mentioned Example 1 (9) was dissolved in 0.1 M acetate buffer (pH 4.5) and digested with 220 µg of pepsin for 10 hr. at 37°C. The reaction mixture was applied to a Sephadex G-100 column (1.0 x 47 cm, Pharmacia), and eluted with 0.1 M borate buffer (pH 8.0) at a flow rate of 4.8 ml/hr. The eluate was recovered in 1 ml portions, and the fractions 16-22 were passed through an Affigel Protein A column (2.0 ml, Bio-Rad). The eluate was concentrated by means of Amicon Ultra-filtrator (PM-10) (Amicon), and a 0.1 M phosphate buffer (pH 6.1) was added thereto. The mixture was concentrated again to give 1.5 ml of F(ab′)₂ fragment (OD₂₈₀ 0.850). To the fragment was added 150 µl of 0.1 M 2-mercaptoethylamine, and the mixture was incubated at 37°C for 120 min., applied to a Sephadex G-25 column (1.0 x 51.5 cm, Pharmacia), and eluted with 0.1 M phosphate buffer (pH 6.1) at a flow rate of 24 ml/hr. Fractions 15-25 were concentrated by Amicon Ultro-filtrator PM-10 (Amicon) to give 0.5 ml of Fab′ fragment.

### Preparation of maleimido-POD

Peroxidase (POD) (3.0 mg, Sigma) was dissolved in 0.3 ml of 0.1 M phosphate buffer (pH 6.1), to which was added 30 µl of a solution of 3.2 mg of N-succinimidyl 4-(N-maleimidomethyl)cyclohexane-1-carboxylate (Pierce) dissolved in 40 µl of dimethylformamide, and the mixture was incubated at 30°C for 60 min. The reaction mixture was centrifuged at 3000 rpm for 10 min., and the supernatant was applied to a Sephadex G-25 column (1.4 x 41.0 cm, Pharmacia), and eluted with a 0.1 M phosphate buffer (pH 6.1) at a flow rate of 18 ml/hr. The eluate was recovered in 1 ml portions, and the fractions 13-17 were concentrated to 0.5 ml using Amicon Ultra-filtrator PM-10, whereby maleimido-POD was obtained.

### Preparation of POD-labeled Fab′ fragment of anti-PSTI antibody

The above-mentioned maleimido-POD (0.5 ml) was mixed with Fab′ fragment (0.5 ml), and the mixture was allowed to stand for 16 hr. at 4°C. The mixture was then applied to an Ultrogel AcA 44 column (1.6 x 55.5 cm, LKB Produkter AB), and eluted with a 0.1 M phosphate buffer (pH 6.5) at a flow rate of 13 ml/hr. The eluate was recovered in 1.0 ml portions. POD-labeled Fab′ fragment of anti-PSTI monoclonal antibody was obtained from the fractions 48-53 (6.0 ml).

### Preparation of beads sensitized by anti-PSTI monoclonal antibody 150

Two hundred and fifty-two pieces of bead (0.18 g per 10 polystyrene beads, dia. 3 mm, Wako Pure Chemicals) were immersed in 20 ml ( 15.5 µg/ml) of a solution of anti-PSTI monoclonal antibody 150 dissolved in 0.1 M tris-hydrochloric acid buffer (pH 8.5), and allowed to stand for 24 hr. at 4°C. The beads were then washed 4 times with Buffer A (0.1 M phosphate buffer, pH 7.0, containing 0.1% bovine serum albumin, 0.3 M sodium chloride, 1 mM magnesium chloride, and 0.1% sodium azide), to obtain the beads carrying anti-PSTI monoclonal antibody 150.

### Two-step measurement

A piece of bead carrying anti-PSTI monoclonal antibody 150 was placed in one well of a 96-well microplate. To the well was added 250 µl of Buffer A, and allowed to stand for 10 min. at room temperature. The buffer was then removed by suction, and 200 µl of Buffer A and 20 µl of a test sample or a standard PSTI were added thereto, and the mixture was allowed to stand for 90 min. at room temperature. The bead was then washed 4 times, each with 250 µl of Buffer B (0.01 M phosphate buffer, pH 7.0, containing 0.1% bovine serum albumin), after which 200 µl of POD-labeled Fab′ fragment of anti-PSTI antibody (diluted to 1:100 with Buffer B) was added, and the mixture was allowed to stand for 90 min. at room temperature. After removing the buffer by suction, the bead was washed 4 times, each with 250 µl of Buffer B. The bead was transferred to a well of another 96-well microplate. To the well, 3 mg/ml of ortho-phenylenediamine (OPD) and 200 µl of Mclvaine buffer (pH 6.5) containing 0.02% hydrogen peroxide were added. After incubating the mixture for 20 min. at room temperature, 50 µl of 1 N sulfuric acid was added to stop the enzyme reaction. The concentration of PSTI was determined by measuring the absorbance of the mixture at 492 nm.

### One-step measurement

A piece of bead carrying anti-PSTI monoclonal antibody 150 was placed in a well of a 96-well microplate. Twenty µl of a test sample or a standard PSTI was added thereto, followed by the addition of 200 µl of POD-labeled Fab′ fragment of anti-PSTI antibody (diluted to 1:100 with Buffer B). The plate was allowed to stand for 120 min. at room temperature, after which the buffer was removed by suction, and the bead was washed 4 times, each with 250 µl of Buffer B. The bead was then transferred to a well of another 96-well microplate. To the well was added 200 µl of 0.1 M Mclvaine buffer containing 0.3 mg/ml of OPD, which was incubated for 20 min. at room temperature. Fifty µl of 1 N sulfuric acid was then added to stop the enzyme reaction. The concentration of PSTI was measured with absorbance at 492 nm.

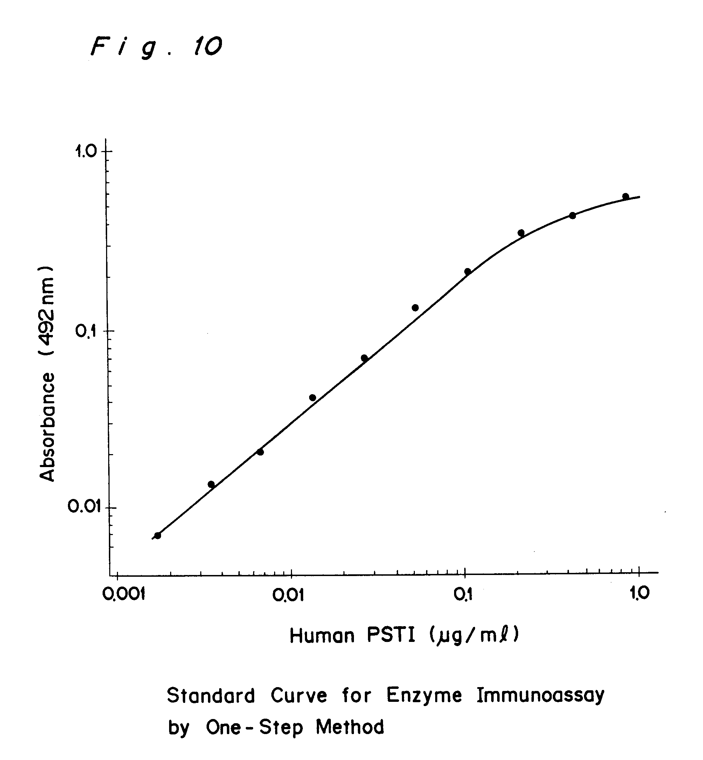

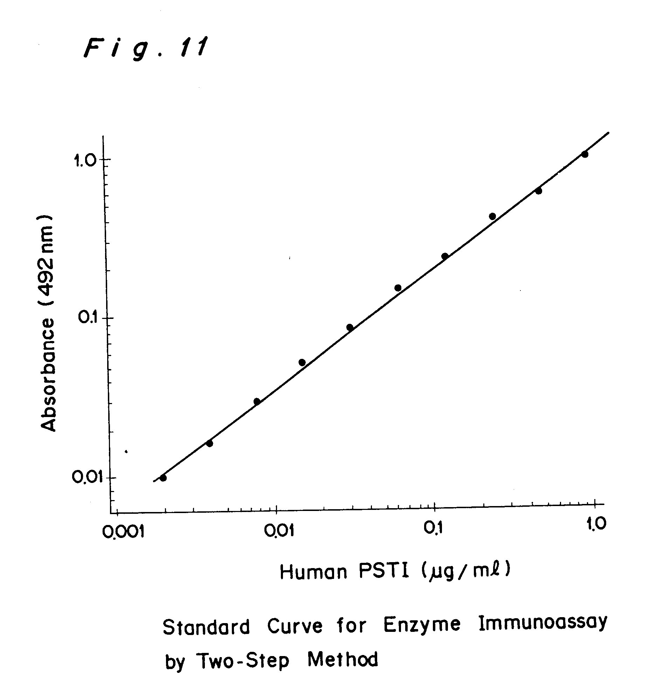

The results of the experiments are shown in Figs. 10 and 11, which show that the above methods allow a quantitative measurement of PSTI.

## Claims

1. A monoclonal antibody obtainable from the hybridomas FERM BP 1583, FERM BP 1584 or FERM BP 1585.

2. The hybridoma deposited under the accession numbers FERM BP 1583, FERM BP 1584 or FERM BP 1585.

3. A method for the determination of human PSTI which comprises measuring human PSTI by immunoassay using at least one monoclonal antibody as defined in claim 1.

4. A method as claimed in claim 3 wherein the immunoassay is radioimmunoassay or enzyme immunoassay.

5. The use of a monoclonal antibody as defined in claim 1 in an in vitro diagnostic procedure.

6. A method of producing a hybridoma as defined in claim 2 or an antibody as defined in claim 1 comprsising immunizing an animal with human PSTI and obtaining antibody-secreting cells therefrom, fusing said cells with myeloma cells, and selecting from the resulting fusion product capable of secreting a monoclonal antibody which recognizes hum PSTI, and optionally culturing the selected fusion product to produce the desired monoclonal antibody.

## Patentansprüche

1. Monoclonaler Antikörper, erhältlich aus den Hybridomen FERM BP 1583, FERM BP 1584 oder FERM BP 1585.

2. Hybridom, hinterlegt unter der Hinterlegungsnummer FERM BP 1583, FERM BP 1584 oder FERM BP 1585.

3. Verfahren zur Bestimmung von menschlichem PSTI, umfassend das Messen von menschlichem PSTI durch einen Immunoassay unter Verwendung von mindestens einem monoclonalen Antikörper nach Anspruch 1.

4. Verfahren nach Anspruch 3, wobei der Immunoassay ein Radioimmunoassay oder Enzymimmunoassay ist.

5. Verwendung eines monoclonalen Antikörpers nach Anspruch 1 in einem in vitro-Diagnoseverfahren.

6. Verfahren zur Herstellung eines Hybridoms nach Anspruch 2 oder eines Antikörpers nach Anspruch 1, umfassend das Immunisieren eines Tieres mit menschlichem PSTI und Gewinnung von Antikörper-sekretierenden Zellen daraus, Fusion der Zellen mit Myelomzellen, Auswählen eines Hybridoms aus dem erhaltenen Fusionsprodukt, das zur Sekretion eines menschliches PSTI erkennenden monoclonalen Antikörpers fähig ist, und gegebenenfalls Züchtung des ausgewählten Fusionsprodukts zur Produktion des gewünschten monoclonalen Antikörpers.

## Revendications

1. Anticorps monoclonal pouvant être obtenu à partir des hybridomes FERM BP 1583, FERM BP 1584 ou FERM BP 1585.

2. Hybridome déposé sous le numéro d'accès FERM BP 1583, FERM BP 1584 ou FERM BP 1585.

3. Méthode de dosage de l'inhibiteur de trypsine sécrété par le pancréas humain (ITSP humain), qui comprend la mesure de l'ITSP humain par un dosage immunologique utilisant au moins un anticorps monoclonal tel que défini dans la revendication 1.

4. Méthode selon la revendication 3 dans laquelle le dosage immunologique est un dosage radio-immunologique ou un dosage immuno-enzymatique.

5. Utilisation d'un anticorps monoclonal tel que défini dans la revendication 1 dans un procédé de diagnostic in vitro.

6. Méthode de production d'un hybridome tel que défini dans la revendication 2 ou d'un anticorps tel que défini dans la revendication 1, selon laquelle on immunise un animal avec de l'ITSP humain et on en obtient des cellules qui sécrètent l'anticorps, on fusionne lesdites cellules avec des cellules de myélome, et on sélectionne à partir du produit obtenu un produit de fusion capable de sécréter un anticorps monoclonal qui reconnaît l'ITSP humain, et éventuellement on cultive le produit de fusion sélectionné pour produire l'anticorps monoclonal désiré.
